# EUROPÄISCHE PATENTSCHRIFT

(11) **EP 0 833 587 B1**
(45) Veröffentlichungstag und Bekanntmachung des Hinweises auf die Patenterteilung: **22.10.2003**
(21) Anmeldenummer: 97916354.0
(22) Anmeldetag: 21.03.1997
(51) Int. Cl.: A61B 5/117

(54) **VERFAHREN ZUR PERSONENIDENTIFIKATION**
METHOD OF IDENTIFYING PEOPLE
PROCEDE D'IDENTIFICATION DE PERSONNES

(30) Priorität: 10.04.1996 DE 19614220
(43) Veröffentlichungstag der Anmeldung: 08.04.1998
(73) Patentinhaber: Infineon Technologies AG, 81669 München (DE)
(72) Erfinder: ECCARDT, Peter-Christian, D-85521 Ottobrunn (DE); VOSSIEK, Martin, D-80789 München (DE)
(74) Vertreter: Epping Hermann & Fischer
(86) Internationale Anmeldenummer: DE9700585
(87) Internationale Veröffentlichungsnummer: WO97037592

(56) Entgegenhaltungen:
- EP-A- 0 197 810
- EP-A- 0 619 095
- WO-A-96/25099
- FR-A- 2 406 986

## Beschreibung

Die Erfindung betrifft ein Verfahren zur Personenidentifikation mittels Ultraschall.

Aus dem Stand der Technik US 4 564 019 ist ein Verfahren zur Messung von Charakteristika lebenden Gewebes durch Ultraschall bekannt. Aus der DE 4 226 865 A1 ist ein Ultraschalldiagnosegerät für die Dermatologie bekannt.

Bildgebende Verfahren zur Erkennung der Leistenstruktur der Hautoberfläche sind aus der US 5 224 174 und der WO 95/12354 bekannt. Aus der WO 94/25938 ist ein Verfahren zur Personenidentifikation bekannt, welches ebenfalls die Position der Schweißporen erfaßt. Aus der US 4 977 601, der EP 0 619 095 A1, der US 5 218 644, der US 5 258 922, der EP 0 402 779 A2 und der WO 94/24937 sind Verfahren bekannt, die den Betrag des von einer Wandlerstrecke gewonnenen und über mehrere Wandlerstrecken gemittelten Ortsfrequenzspektrum der Leistenstruktur oder anderer in den Anmeldungen nicht näher beschriebenen Zellstrukturen ("Epithelstruktur") zur Personenidentifikation heranziehen.

Aus der WO 94/01043 ist ein Verfahren bekannt, das Ultraschallwellen mit elektromagnetischen Wellen kombiniert.

Aus der WO 95/06262 ist ein Verfahren bekannt, das mit inverser Anregung arbeitet.

Die Aufgabe der Erfindung ist es, ein Verfahren zur Personenidentifikation anzugeben, das eine sichere Personenidentifikation gewährleistet und einfach zu realisieren ist.

Die Aufgabe wird durch ein Verfahren gemäß dem Patentanspruch 1 gelöst.

Vorteilhafte Weiterbildungen ergeben sich aus den abhängigen Ansprüchen.

Die Erfindung wird anhand von drei Figuren weiter beschrieben.

Die Oberhaut der menschlichen Fingerkuppe, auch als Epidermis bezeichnet, besteht aus einer Hornschicht, einer Keimschicht und einer Basalschicht. Die Grenzfläche der Hornschicht zum ankoppelnden Medium stellt für Ultraschall einen hohen Impedanzsprung dar und kann zur Erkennung der Oberfläche herangezogen werden. Die aus dem obengenannten Stand der Technik bekannten bildgebenden Verfahren zur Erkennung der Leistenstruktur der Hautoberfläche nutzen diesen Effekt.

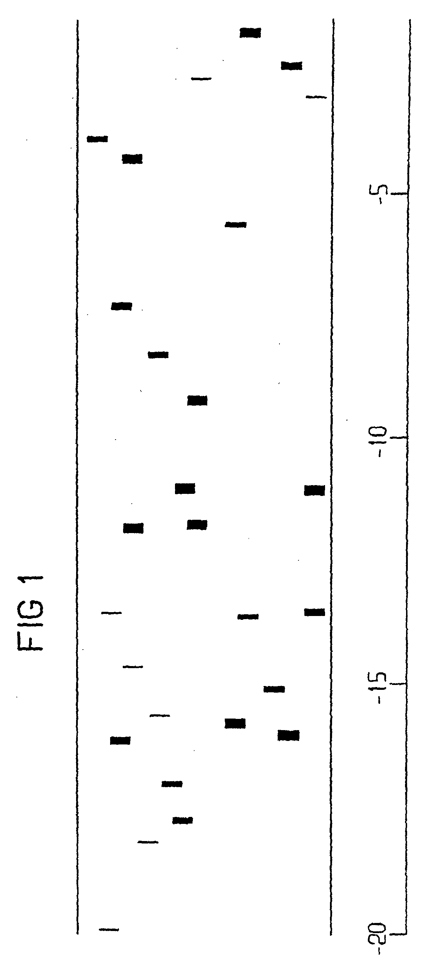

Die Hornschicht ist in ihren akustischen Eigenschaften weitgehend konstant und somit echoarm. Eine Ausnahme bilden die in der Hornschicht vorhandenen Schweißporen, die meist entlang der Furchen zwischen den Leisten angeordnet sind und in einem Schnitt des Ultraschallbildes (C-Scan) in einer bestimmten Tiefe, typischerweise zwischen 100 und 250 um unter der Hautoberfläche erfaßt werden können (vgl. hierzu Figur 1).

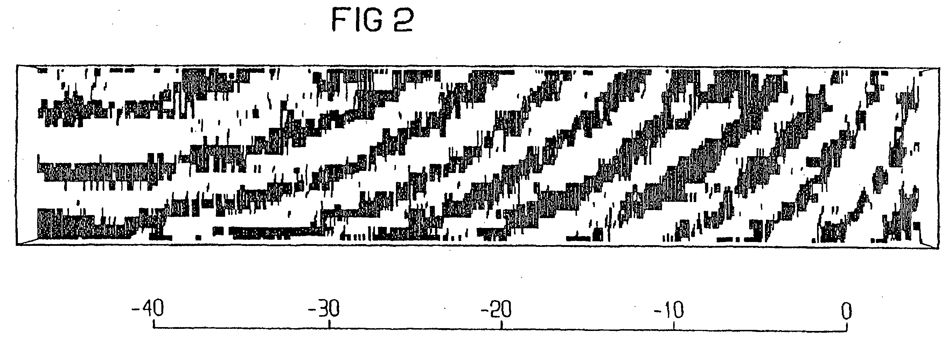

Die an der Fingeroberfläche vorhandene Leistenstruktur wiederholt sich in einer tieferliegenden Hautschicht, typischerweise 200 bis 500 µm (vgl. Figur 2). Der Übergang von der abgestorbenen (wasserarmen) Schicht der Oberhaut zu den wasserhaltigen, wie die Fingerleisten strukturierten hautbildenden Schichten stellt einen deutlich meßbaren Impedanzsprung dar, so daß sich diese Hautschicht ebenfalls zur Selektion von personenspezifischen Merkmalen eignet.

Ein Vorteil der zusätzlichen Detektion der tieferen Hautschichten ist eine höhere Fälschungssicherheit und eine gleichbleibende Erkennungsrate bei einer oberflächlichen Verletzung der Haut. Aufgrund des guten von der Oberfläche der Haut zu erzielenden mit Ultraschall aufgenommenen Bildes wird die Oberflächenstruktur der Haut ständig zur Identifizierung herangezogen. Die zusätzliche Identifizierung aufgrund von tieferliegenden Hautschichten verbessert das Verfahren jedoch wesentlich.

Ein besonderer Vorteil der Detektion der Kanäle von Schweißdrüsen liegt darin, daß die gewonnenen Datensätze wegen der binären Information (Schweißdrüse vorhanden oder nicht vorhanden) mit wenig Speicherbedarf und Rechenzeit verarbeitet werden können.

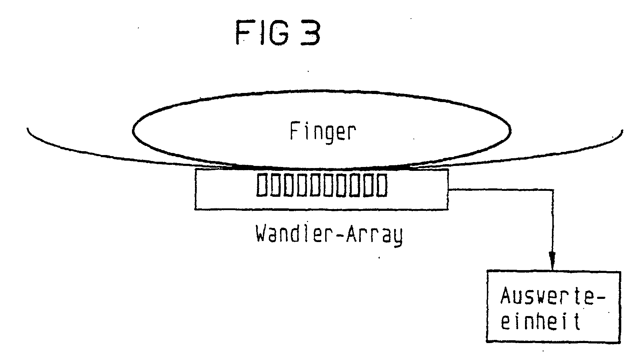

Ein zweidimensionales Ultraschallwandler-Array von ca. 20*20 bis 200*200, typischerweise 100*100 Ultraschallwandlern mit einem Wandlerabstand von ca. 50 bis 200 µm wird so angeordnet, daß die informationstragenden Teile der Fingerkuppe, d.h. der Bereich, in dem auch die an der Hautoberfläche erkennbare Leistenstruktur signifikante Merkmale aufweist, erfaßt werden (vgl. Figur 3). Durch ein Puls-Echo-Verfahren wird die Echoamplitude der zu untersuchenden Hautschicht im Bereich von 100 bis 1000 µm untersucht. Die einzelnen Wandlerstrecken können als Einzelwandlerstrecke (jeder Wandler mißt sein eigenes Echo) oder als getrennte Wandlerstrecken (ein Wandler sendet und ein oder mehrere Wandler empfangen) ausgeführt sein.

Die kostengünstige Realisierung gemäß des vorliegenden Erfindung ergibt sich, wenn die Echoamplituden nur einer bestimmten Tiefe, z.B. die Ebene der Schweißdrüsen oder die Ebene der tieferliegenden informationstragenden Hautschichten, als skalarer oder binärer Wert abgespeichert und einer Mustererkennung zugeführt werden. Zum Ausgleich von unterschiedlichen Hautdicken kann hierbei die Adaption der Meßtiefe notwendig sein.

Die Erfindung benutzt die Schweißdrüsen der Hornschicht der menschlichen Fingerkuppe als personenspezifisches Merkmal. Diese werden mittels einer Ultrallschall-Sende/Empfangseinheit detektiert. Die von der Ultraschall-Sende/Empfangseinheit stammenden Informationen werden in der der Ultraschall-Sende/Empfangseinheit nachgeschalteten Auswerteeinheit weiter verarbeitet.

Die einzelnen Ultraschallwandler des Ultraschallwandlerarrays haben eine laterale Auflösung zwischen 50 und 200 um und können mikromechanisch hergestellt werden.

Es ist auch möglich, 2-2-Composite-Ultraschallwandler mit einer lateralen Auflösung zwischen 50 und 200 µm zu verwenden.

Die Echoamplituden oder die Maxima der Echoamplituden können in einer bestimmten Hautschicht aufgezeichnet und einer Bildverarbeitung zugeführt werden.

## Patentansprüche

1. Verfahren zur Personenidentifikation, bei dem die Person oder die Personen anhand ihrer Schweißdrüsen in der Haut identifiziert werden,
**dadurch gekennzeichnet, dass** die Schweißdrüsen mittels einer Ultraschall-Sende/Empfangseinheit detektiert werden,
dabei ein Puls-Echo-Verfahren eingesetzt wird und Echoamplituden in einer bestimmten Tiefe einer Ebene der Schweißdrüsen im Bereich zwischen 100 µm und 250 µm erfasst und als skalarer oder binärer Wert abgespeichert und einer Mustererkennung zugeführt werden.

2. Verfahren nach Anspruch 1, bei dem
ein zweidimensionales Ultraschallwandler-Array von 20×20 bis 200×200 Ultraschallwandlern mit einem Wandlerabstand von 50 µm bis 200 µm verwendet wird.

3. Verfahren nach Anspruch 2, bei dem
das Ultraschallwandler-Array mit Einzelwandlerstrecken ausgeführt ist und jeder Ultraschallwandler sein eigenes Echo misst.

4. Verfahren nach Anspruch 2, bei dem
das Ultraschallwandler-Array mit getrennten Wandlerstrecken ausgeführt ist und zu einem sendenden Ultraschallwandler ein oder mehrere empfangende Ultraschallwandler vorgesehen sind.

## Claims

1. Method for personal identification, in which the person or the persons are identified with the aid of their sweat glands in the skin, **characterized in that** the sweat glands are detected by means of an ultrasonic transmission/reception unit, where a pulse-echo method is used and echo amplitudes of a specific depth of a plane of the sweat glands in the region between 100 µm and 250 µm are detected and stored in scalar or binary value form and fed to a pattern recognition stage.

2. Method according to Claim 1, in which a two-dimensional ultrasonic transducer array of 20×20 to 200×200 ultrasonic transducers with a transducer separation of 50 µm to 200 µm is used.

3. Method according to Claim 2, in which the ultrasonic transducer array is configured with individual transducer channels and each ultrasonic transducer measures its own echo.

4. Method according to Claim 2, in which the ultrasonic transducer array is configured with split transducer channels and, in addition to a transmitting ultrasonic transducer, one or more receiving ultrasonic transducers are provided.

## Revendications

1. Procédé d'identification de personnes, dans lequel on identifie la personne ou les personnes au moyen de leurs glandes sudoripares de la peau,
**caractérisé en ce que**
on détecte les glandes sudoripares au moyen d'une unité émetteur/récepteur d'ultrasons,
on utilise à cet effet un procédé impulsion-écho et on détecte les amplitudes d'écho à une profondeur déterminée d'un plan des glandes sudoripares de l'ordre de 100 µm à 250 µm que l'on mémorise sous la forme d'une valeur scalaire ou binaire et que l'on envoie à une reconnaissance de modèle.

2. Procédé suivant la revendication 1, dans lequel on utilise un réseau à deux dimensions de transducteurs ultrasonores de 20 × 20 à 200 x 200 transducteurs ultrasonores ayant une distance entre les transducteurs de 50 µm à 200 µm.

3. Procédé suivant la revendication 2, dans lequel on réalise le réseau de transducteurs ultrasonores ayant des zones individuelles de transducteurs et chaque transducteur ultrasonore mesure son propre écho.

4. Procédé suivant la revendication 2, dans lequel on réalise le réseau de transducteurs ultrasonores avec des zones de transducteurs distinctes et pour un transducteur ultrasonore d'émission, il est prévu un ou plusieurs transducteur(s) ultrasonores de réception.
